(19) Europäisches Patentamt
European Patent Office
Office européen des brevets

(11) **EP 1 815 828 B1**

(12) **EUROPEAN PATENT SPECIFICATION**

(45) Date of publication and mention
of the grant of the patent:
**22.10.2014 Bulletin 2014/43**

(51) Int Cl.:
**A61F 5/443** (2006.01)

(21) Application number: **07002257.9**

(22) Date of filing: **02.02.2007**

(54) **Ostomy appliance with recovery resistant moldable adhesive**

Ostomievorrichtung mit formwiederherstellungresistentem, formbarem Kleber

Appareillage stomique comportant un adhésif moulé résistant à la restauration

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.02.2006 US 765349 P**

(43) Date of publication of application:
**08.08.2007 Bulletin 2007/32**

(73) Proprietor: **ConvaTec Technologies Inc.**
**Las Vegas, NV 89169-6754 (US)**

(72) Inventors:
• **Fattman, George F.**
**08054 New Jersey (US)**

• **Sambasivam, Mahesh**
**08534 New Jersey (US)**

(74) Representative: **Mays, Julie et al**
**Venner Shipley LLP**
**200 Aldersgate**
**London**
**EC1A 4HD (GB)**

(56) References cited:
**WO-A-03/026541    WO-A1-98/53772**
**US-A- 5 328 696    US-A1- 2004 102 744**

## Description

FIELD OF THE INVENTION

The present invention relates to an ostomy device and more particularly to an ostomy device having a layer of adhesive that is manually conformable to the shape of a stoma.

BACKGROUND OF THE INVENTION

A variety of health conditions may lead to ostomy surgery, a procedure whereby the intestinal (or urinary) tract is diverted from the rectum (or bladder) to the abdomen where the creation of a stoma permits elimination of waste. Most ostomates are incontinent and wear an appliance to collect waste and protect the peristomal area from its effects. The most commonly used ostomy appliances are typically designed to include a collection bag or pouch and an adhesive component frequently referred to as a wafer or skin barrier. The adhesive wafer attaches the pouch to the abdomen for collection of waste from the stoma. The wafer also protects the peristomal skin from the effluent from the stoma. Protection of peristomal skin is critical to the successful functioning of the device since effluent causes rapid and severe skin break down. In addition to being painful, denuded skin is poorly suited for attachment of subsequent skin barriers, leading to a cycle of poor attachment, reduced skin protection and worsening skin condition.

Critical elements of skin protection are secure, leak-proof adhesion to peristomal skin and a proper fit of the skin barrier to the stoma. More specifically, it is critical to closely match the wafer opening to the stoma perimeter.

The most commonly used adhesives for ostomy appliances are referred to as hydrocolloid adhesives because their constituents include hydrophilic powders that form gels in the presence of moisture. The non-hydrocolloid portion of the formula may be any material that adheres favorably to skin. In order to obtain the most secure attachment, which is necessary for reliable and durable performance of the device, hydrocolloid adhesives for ostomy wafers typically include adhesives that adhere aggressively to skin and that contain large amounts of the powdered hydrocolloids. In that case, proper fit of the wafer to the stoma can be problematic because most commercially available wafers are not manufactured to the identical shape of the stoma and must be modified in some way.

The modification most frequently made to adapt the wafer to a particular stoma is to cut the wafer opening to closely match the shape of the stoma. The edge of the wafer opening should be approximately 3.175 mm (0.125 inches) from the stoma around its entire circumference for optimum fitting of the device. It is difficult to obtain a precise cut of this kind with hydrocolloid wafers because of the aggressive nature of the adhesive and the increased modulus of the adhesive that results from the high concentration of filler (typically at least 30% of hydrocolloid powder by weight) required for secure adhesion.

Reference is made to patents GB2290974 and U.S. Patent No. 6,840,924, both of which describe wafer designs that alleviate problems associated with cutting ostomy adhesives. These designs enable the wafer opening, sometimes referred to as a starter hole, to be pliably shaped or molded with the fingers to obtain the desired custom match of the wafer opening to the shape of the stoma. GB2290974 claims the adhesive as a moldable mass of substantially non-memory, putty-like adhesive. The required lack of memory eliminates the possibility that the adhesive will recover its original shape after molding. However, this lack of memory results in the disadvantage that the adhesive may creep (flow) under ambient conditions or during use. It would improve this adhesive to add memory through crosslinking its components.

Adhesion strength and durability of ostomy adhesives can be greatly improved by chemically and/or physically crosslinking one or more of the components comprising the non-hydrocolloid portion of the adhesive. An example of one method to add physical or mechanical crosslinks would be incorporation of styrene-isoprene-styrene block copolymer into the non-hydrocolloid portion of the adhesive. Styrene copolymer technology is well known in adhesives formulation. Chemical crosslinks could be introduced to the non-hydrocolloid portion of the formulation by addition of a functionalized or substituted material capable of forming covalent or ionic bonds between itself and / or with at least one other material in the non-hydrocolloid portion of the formulation. Crosslinks may also be imparted to the adhesive by irradiation from an energy source, for example, heat, gamma irradiation, or ultraviolet light, that is capable of forming bonds between one or more components of the formulation.

Incorporation of crosslinks introduces cohesive strength and elasticity into the adhesive. For a moldable adhesive this elasticity causes it to remember its unmolded shape and to tend to recover that shape. For a molded ostomy wafer to recover some or all of its shape before molding would be incompatible with proper fitting of the device to the stoma. Furthermore, incorporation of crosslinks into the adhesive may cause some adhesives to become more difficult to mold in that greater force is required to shape the adhesive.

U.S. Patent No. 6,840,924 discloses exposing adhesive on the pouch side of the wafer. This design enables adhesives with components capable of shape memory to be tacked down to the pouch side of the wafer, for example, by rolling the adhesive wafer back upon itself from the body side. Thus, no recovery of shape should be possible for cases where the adhesive strength of the bond formed by adhering the two surfaces exceeds the force driving the adhesive to recover its original shape. These recovery forces may include, for example, the tensile strength or yield strength of the adhesive or may be characterized by the compliance of the adhesive.

One advantage of this design is that it overcomes the difficulty of adhering molded adhesive with elasticity to low surface energy backing materials that are typically laminated to the side of the adhesive facing away from the body (pouch side).

Ostomy adhesive backing materials are typically films, foams or fabrics comprised of polyethylene and its copolymers, or other olefinic and non-olefinic polymers that are not especially tacky, have low surface energy, and are not easily adhered to. These laminates or backings are used to prevent adhesion of the pouch side of the adhesive to other objects, for example, the clothing of the wearer. They also protect the wafer from external attack, for example, stomal effluent, water from a shower or swimming pool, etc.

U.S. Patent No. 4,831,070 discloses moldable elastic pressure sensitive adhesives that are solventless and can be molded and cured at room temperature and which are useful for making medical adhesives, e.g., those used for sealing ostomy devices. It is believed that the invention described is an ostomy seal, a very specific product in the ostomy business used between an adhesive wafer and a pouch or between the skin and an ostomy wafer for improved device security. Commercially available examples include Eakin Seals, Microderm washers, Softflex Rings, Osto-seals, and many others. One example where it is believed that this patent describes an ostomy seal that acts as a filler between the body and the attached ostomy device may be found in column 8, line 40: For example, if a body of the cured elastomeric pressure sensitive adhesive is to be used to form a seal between the stoma of an ostomy patient and an attached appliance, the procedure would be to (a) form a body of elastomeric pressure sensitive adhesive using the composition as described herein, (b) attach the elastomeric pressure sensitive adhesive body on a patient's skin around the stoma, and (c) attach the appliance to the elastomeric pressure sensitive adhesive body.

Reference may be made to US-A-2004/102744 which describes the precharacterizing features of the present invention. Reference may also be made to WO-A-03/026541 and WO 98/53772.

It would be desirable to yet further improve the versatility of an ostomy appliance using a moldable or shapeable adhesive.

SUMMARY OF THE INVENTION

The present invention is defined in the claims.

The preferred embodiment illustrates a moldable ostomy wafer comprised of a cross-linked silicone adhesive having an elastic modulus in shear (G') between about $1\times10^4$ and $1\times10^7$ Pascal when measured at 25°C and at shear rates between about 1 and 100 reciprocal seconds and capable of forming a bond to a wafer backing sufficient in strength to limit shape recovery to less than 3.175 millimetres (0.125 inches) from the molded dimension. The ostomy wafer may be a component of an ostomy appliance having an integral pouch and wafer design (i.e., a one piece design) or a separable pouch and wafer design (i.e., a two piece design). Molding of the adhesive may be accomplished easily by the simple application of finger pressure to fold, roll, stretch or otherwise shape the adhesive to a new and substantially stable dimension.

DETAILED DESCRIPTION OF THE INVENTION

Silicone pressure sensitive adhesives are typically comprised of two major components, a siloxane polymer and a silicate resin. The siloxane polymers have alternating silicone and oxygen atoms along their main chain. They cover a wide range of molecular weights, ranging from about 170 g/mol to more than 1,000,000 g/mol. Examples of polymers used in silicone adhesives include polydimethylsiloxane, polymethylphenylsiloxane, polydimethyldiphenylsiloxane, and other silicone polymers including various organosiloxanes that are described generally as polysiloxanes. Silicones are generally hydrophobic, but they can be made less hydrophobic or more hydrophilic by modifying or copolymerizing them, for example, with alkylene oxides. Silicones and silicone adhesives are referred to herein interchangeably with the term polysiloxanes. An example of a silicate resin is tetrakis (trimethylsiloxy) silicate.

The cross-linked silicone adhesive may be comprised of a polyorganosiloxane and a silicate resin having a functionality of at least two sites capable of reacting with the polyorganosiloxane, optimally including plasticizers, for example, silicone oils including polydimethylsiloxane, optionally, also including non-hydrophilic organic or inorganic fillers, for example, amorphous precipitated silica as needed to obtain elastic modulus in shear (G') between about $1\times10^4$ and $1\times10^7$ Pascal when measured at 25°C and at shear rates between about 1 and 100 reciprocal seconds.

Alternatively, gels of silicone may also be used as the adhesive component of the device. Silicone gels are generally formed from linear or branched silicone polymer having reactive groups thereon, as is known in the art. Such reactive groups undergo a crosslinking reaction during curing. Examples of crosslinking reactions include the hydrosilylation reaction in which a silicone having an Si-H reactive group reacts with a silicone having an aliphatically unsaturated reactive group in the presence of a platinum or rhodium catalyst. Alternatively, the reaction can involve the reaction of a silicone having an Si-OH reactive group with a silicone or a chain extender (e.g., a silane) having an alkoxy reactive group in the presence of a metal catalyst. A third possible gel may be formed from a silicone having an Si-OH containing polymer that is mixed with an alkoxysilane in the presence of a titanate catalyst.

In a preferred form of the invention, the adhesive is a polysiloxane available commercially under the trade name Bio-PSA from Dow Corning including the grade series Bio-PSA 7-4XXX. Suitable grades include 7-4101, 7-4102, 7-4103, 7-4201, 7-4202, 7-4203, 7-4301, 7-4302, 7-4303, 7-4401, 7-4402, 7-4403, 7-4501, 7-4502, 4-4503, 7-4601, 7-4602, 7-4603. These are one-part, adhesives that are cured (crosslinked) by the supplier and commercially available in a suitable solvent. Optionally, additional fillers or plasticizers may be added. In a preferred form of the invention, the adhesive is Bio-PSA 7-4560, a one part, pre-cured (crosslinked) adhesive that is essentially solvent free and referred to as a hot melt adhesive. A more preferred adhesive formulation is obtained by blending Bio-PSA 7-4560 with powdered silica filler to adjust elastic modulus and so to also modify adhesion strength. Optionally, plasticizers including, for example, silicone oils like polydimethylsiloxane may be added to improve moldability. Suitable formulations are believed to include up to about 50% silica or other fillers optionally including up to about 25% plasticizer.

The ostomy wafer of the invention has a backing opposite its body facing surface and covering substantially that entire opposite surface. The backing should have sufficient compliance that it will not inhibit unrecoverable shaping of the adhesive. The thickness of the backing may be between about 0.0127 mm and 6.35 mm (0.0005 inch and 0.25 inch). The backing may be moisture vapor permeable, as in the case of a porous, perforated or otherwise discontinuous structure, comprised of water vapor permeable polymers including, for example, polyurethanes, polyethers, polyesters and polyamides, or combinations thereof. Alternatively, the backing may be moisture impermeable, for example, a continuous sheet of an olefinic polymer. The backing may also take the form of a foam, meshes, film or woven or non-woven fabric or a sheet of any polymeric or inorganic material with sufficient compliance that it will not inhibit unrecoverable shaping of the adhesive. The backing may also include either singly or in layers any planar or non-planar covering of or attachment to the adhesive that is not a pressure sensitive adhesive, e.g., that does not pressure sensitively adhere substantially to skin or clothing.

The invention has the following benefits over the prior art:

    a. improved moldability;
    b. more stable geometry after shaping but with good skin adhesion;
    c. stable geometry after exposure to stomal effluent;
    d. improved skin protection;
    e. excellent skin adhesion;
    f. good biocompatibility;
    g. resistant to stomal effluent; and
    h. improved adhesion to low surface energy backings.

Because the cost of silicone adhesives is generally greater than other adhesives, it is desirable to minimize its use in the device by confining it to a thin layer next to the skin. However, it is known that the adhesion strength or security of a skin contacting adhesive can benefit greatly from a certain amount of rigidity which helps to keep the device in place during wear. The amount of rigidity should not exceed what would become uncomfortable to the wearer. To address these issues, a thin layer of silicone adhesive may be layered onto a thicker more rigid backing surface, the thickness and rigidity being limited by the comfort of the wearer and also by the ease of molding the device. Backings or backing surfaces would, preferably, include meshes, films, open or closed cell foams, sheets, or open or closed cell foams, a non-adhesive coating, and combinations thereof.

Alternatively, rigidity in the adhesive may be imparted to the device by using a thicker adhesive layer in the construction together with a thin backing layer. The adhesive may be crosslinked to obtain the desired rigidity, or a lightly crosslinked adhesive may be reinforced to the desired rigidity by incorporation of additional components into its bulk. Backings would preferably include films, open or closed cell foams, non-woven and woven fabrics, and combinations thereof.

Example 1

A 0.025 mm (0.001 inch) thick film of silicone adhesive Bio-PSA 7-4501 is prepared by pouring the adhesive onto a suitable sheet of release liner, drawing a blade across the liner surface to coat it with the adhesive, inserting the coated liner into an oven for removal of solvent, removing the adhesive from the oven, laminating it with a 0.381 mm (0.015 inch) thick foam using sufficient pressure to securely attach the foam backing, and cutting the laminated adhesive sheet into the shape of an ostomy device for attachment to the skin. The foam of the example is available from Voltek LLC under the trade name Volara EO. Prior to attachment to the body the adhesive may be manually molded to match the shape of the stoma of the device wearer.

Example 2

A 6.35 mm (0.250 inch) thick adhesive sheet is prepared by addition of Bio-PSA 7-4560 to a heated mixer containing double sigma blades for mixing high viscosity materials. To the heated silicone is added 15% by weight of a filler that is mixed into the silicone to obtain an elastic modulus in shear (G') between about $1 \times 10^4$ and $1 \times 10^7$ Pascal when measured at 25°C and at shear rates between about 1 and 100 reciprocal seconds. The compounded adhesive is removed from the mixer and pressed into a plaque of the desired thickness between two sheets of release liner. One sheet of release liner is then removed and the exposed surface laminated with a thin polyurethane film with a high moisture vapor transmission rate commercially available under the trade name Inspire from Intelicoat. The laminated plaque is cut to the dimensions desired for an ostomy device. Prior to attachment to the body the adhesive may be manually molded to match the shape of the stoma of the device wearer.

Example 3

A 1.9 mm (0.075 inch) thick adhesive sheet is prepared by addition of Bio-PSA 7-4560 to a heated mixer containing double sigma blades for mixing high viscosity materials. To the heated silicone is added 15% by weight of a suitable elastomeric material that is mixed into the silicone to obtain an elastic modulus in shear (G') between about $1 \times 10^4$ and $1 \times 10^7$ Pascal when measured at 25°C and at shear rates between about 1 and 100 reciprocal seconds. The compounded adhesive is removed from the mixer and pressed into a plaque of the desired thickness between two sheets of release liner.

One sheet of release liner is then removed and the exposed surface laminated with an open cell polyethylene foam. The laminated plaque is cut to the dimensions desired for an ostomy device. Prior to attachment to the body the adhesive may be manually molded to match the shape of the stoma of the device wearer.

Example 4

A silicone gel is produced by crosslinking a silicone having Si-H reactive groups and reacting it with silicone having an aliphatically unsaturated reactive group in the presence of a platinum catalyst. The gel produced is cured at 140°C for one hour to obtain an elastic modulus in shear (G') between about $1 \times 10^4$ and $1 \times 10^7$ Pascal when measured at 25°C and at shear rates between about 1 and 100 reciprocal seconds. The gel is laminated with a film/non-woven fabric composite backing material.

**Claims**

1. An ostomy device having a body side surface and an opposing backing surface wherein said body side surface includes a layer of pressure sensitive cross-linked silicone adhesive for attachment to the body, **characterized by** said adhesive being capable of being manually molded to conform to the shape of a stoma, said adhesive having an elastic modulus in shear (G') between $10^4$ and $10^7$ Pascal when measured at 25°C and at shear rates between 1 and 100 reciprocal seconds, and said adhesive being adhereable to said backing surface with a bond strength sufficient to limit shape recovery of the adhesive to less than 3.175 millimeters (0.125 inches) from the molded dimension.

2. The ostomy device of claim 1, wherein said opposing backing surface has a surface that is not a pressure sensitive adhesive.

3. The ostomy device of claim 1, wherein said pressure sensitive cross linked silicone adhesive is a hotmelt silicone adhesive.

4. The ostomy device of claim 1, 2 or 3, wherein said opposing backing surface has sufficient compliance so as to allow reshapeability.

5. The ostomy device of any preceding claim, wherein said opposing backing surface is composed of a material selected from the group consisting of materials from natural or synthetic origin including cellulose, alginates, chitosan and its derivatives, elastomers, polyesters, polyacrylonitriles, polyolefins, diene elastomers, polyamides, polyurethanes, polyethers, polyvinyl alcohol, polyether block amides, polyimides, rayon, robber, silicones, thermoplastic polymers and elastomers, polyacrylates, polymethacrylates, ionomers, polyvinylacetate and its copolymers, polyvinyichloride, polyvinylidene chloride, fluorinated polymers, and combinations thereof.

6. The ostomy device of any preceding claim, wherein said opposing backing surface has a form selected from the group consisting of film, open or closed cell foam, a non-adhesive coating, woven fabric, non-woven fabric, fibers, meshes, screens, wicking structures, perforated structures, porous structures, planar or non-planar sheets, planar or non-planar structures, and combinations thereof.

7. The ostomy device of any preceding claim, wherein the adhesive is a layered structures.

8. The ostomy device of any preceding claim, wherein the backing is a layered structures.

9. The ostomy device of any preceding claim further comprising a release liner to protect the adhesive surface prior to use.

10. The ostomy device of claim 8, wherein the device is a non-planar structure including concave or convex shaped structures.

11. The ostomy device of claim 8, wherein the device is substantially a planar structure.

12. The ostomy device of any preceding claim, wherein said pressure sensitive cross-linked silicone adhesive is a solvent based adhesive.

13. The ostomy device of any preceding claim, wherein said pressure sensitive cross-linked silicone adhesive is a silicone gel adhesive.

14. The ostomy device of any preceding claim, wherein said pressure sensitive cross-linked silicone adhesive is curable silicone adhesive.

**Patentansprüche**

1. Ostomievorrichtung mit einer körperseitigen Oberfläche und einer gegenüberliegenden Trägeroberfläche, wobei die körperseitige Oberfläche eine Schicht aus einem haftklebenden vernetzten Silikonklebstoff zum Befestigen am Körper umfasst, **dadurch gekennzeichnet, dass** der Klebstoff manuell geformt werden kann, um der Form eines Stomas zu entsprechen, wobei der Klebstoff, gemessen bei 25 °C und bei Scherungsraten von 1 bis $\frac{1}{100}$ pro Sekunde, einen elastischen Schermodul (G') zwischen $10^4$ und $10^7$ Pascal hat, und wobei der Klebstoff mit einer ausreichenden Bindungsstärke so mit der Trägeroberfläche verbunden werden kann, dass die Formwiederherstellung des Klebstoffs von dem geformten Maß auf weniger als 3,175 mm (0,125 Zoll) beschränkt werden kann.

2. Ostomievorrichtung nach Anspruch 1, wobei die gegenüberliegende Trägeroberfläche eine Oberfläche hat, die nicht aus einem haftklebenden Klebstoff besteht.

3. Ostomievorrichtung nach Anspruch 1, wobei der haftklebende vernetzte Silikonklebstoff ein Silikonschmelzklebstoff ist.

4. Ostomievorrichtung nach Anspruch 1, 2 oder 3, wobei die gegenüberliegende Trägeroberfläche ausreichend nachgiebig ist, um erneutes Formen zu ermöglichen.

5. Ostomievorrichtung nach einem der vorhergehenden Ansprüche, wobei die gegenüberliegende Trägeroberfläche aus einem Material besteht, das ausgewählt ist aus der Gruppe bestehend aus Materialien natürlichen oder synthetischen Ursprungs, einschließlich Cellulose, Alginaten, Chitosan und seinen Derivaten, Elastomeren, Polyestern, Polyacrylnitrilen, Polyolefinen, Dienelastomeren, Polyamiden, Polyurethanen, Polyethern, Polyvinylalkohol, Polyetherblockamiden, Polyimiden, Viskose, Gummi, Silikonen, thermoplastischen Polymeren und Elastomeren, Polyacrylaten, Polymethacrylaten, Ionomeren, Polyvinylacetat und seinen Copolymeren, Polyvinylchlorid, Polyvinylidenchlorid, fluorierten Polymeren sowie Kombinationen aus diesen.

6. Ostomievorrichtung nach einem der vorhergehenden Ansprüche, wobei die gegenüberliegende Trägeroberfläche eine Form hat, ausgewählt aus der Gruppe bestehend aus offenzelligem oder geschlossenzelligem Schaumstoff, einer nichtklebenden Beschichtung, Gewebe, Vliesstoff, Fasern, Netzen, Sieben, Strukturen mit Dochtwirkung, perforierten Strukturen, porösen Strukturen, ebenen oder nichtebenen Tüchern, ebenen oder nichtebenen Strukturen oder Kombinationen aus diesen.

7. Ostomievorrichtung nach einem der vorhergehenden Ansprüche, wobei der Klebstoff einen geschichteten Aufbau hat.

8. Ostomievorrichtung nach einem der vorhergehenden Ansprüche, wobei der Träger einen geschichteten Aufbau hat.

9. Ostomievorrichtung nach einem der vorhergehenden Ansprüche, ferner einen Abdeckträger umfassend, um die Klebstoffoberfläche vor dem Gebrauch zu schützen.

10. Ostomievorrichtung nach Anspruch 8, wobei die Vorrichtung eine nichtebene Konstruktion ist, einschließlich konkav oder konvex geformter Konstruktionen.

11. Ostomievorrichtung nach Anspruch 8, wobei die Vorrichtung eine im Wesentlichen ebene Konstruktion ist.

12. Ostomievorrichtung nach einem der vorhergehenden Ansprüche, wobei der haftklebende vernetzte Silikonklebstoff ein Lösungsmittelklebstoff ist.

13. Ostomievorrichtung nach einem der vorhergehenden Ansprüche, wobei der haftklebende vernetzte Silikonklebstoff ein Silikongelklebstoff ist.

14. Ostomievorrichtung nach einem der vorhergehenden Ansprüche, wobei der haftklebende vernetzte Silikonklebstoff ein härtbarer Silikonklebstoff ist.

**Revendications**

1. Dispositif de stomie doté d'une surface côté corps et d'une surface de soutien opposée dans lequel ladite surface côté corps comprend une couche d'adhésif autocollant en silicone réticulé pour une fixation au corps, **caractérisé en ce que** ledit adhésif est capable d'être moulé manuellement pour se conformer à la forme d'une stomie, ledit adhésif a un module d'élasticité en cisaillement (G') entre $10^4$ et $10^7$ Pascal lorsqu'il est mesuré à 25 °C et à des vitesses de cisaillement entre 1 et 100 secondes inverses, et ledit adhésif pouvant adhérer à ladite surface de soutien avec une force de liaison suffisante pour limiter une recouvrance de forme de l'adhésif à moins de 3,175 millimètres (0,125 pouce) de la dimension moulée.

2. Dispositif de stomie selon la revendication 1, dans lequel ladite surface de soutien opposée a une surface qui n'est pas un adhésif autocollant sensible à la pression.

3. Dispositif de stomie selon la revendication 1, dans lequel ledit adhésif autocollant en silicone réticulé sensible à la pression est un adhésif thermofusible en silicone.

4. Dispositif de stomie selon la revendication 1, 2 ou 3, dans lequel ladite surface de soutien opposée a une souplesse suffisante de façon à permettre une capacité de remise en forme.

5. Dispositif de stomie selon l'une quelconque des revendications précédentes, dans lequel ladite surface de soutien opposée est composée d'un matériau choisi dans le groupe consistant en des matériaux d'origine naturelle ou synthétique incluant la cellulose, les alginates, le chitosan et ses dérivés, les élastomères, les poly(esters), les poly(acrylonitriles), les poly(oléfines), les élastomères diène, les poly(amides), les poly(uréthanes), les poly(éthers), le poly(alcool vinylique), les amides à séquence poly(éther), les poly(imides), la rayonne, le caoutchouc, les silicones, les polymères et élastomères thermoplastiques, les poly(acrylates), les poly(méthacrylates), les ionomères, le poly(acétate de vinyle) et ses copolymères, les poly(chlorure de vinyle), le poly(chlorure de vinylidène), les polymères fluorés, et leurs combinaisons.

6. Dispositif de stomie selon l'une quelconque des revendications précédentes, dans lequel ladite surface de soutien opposée a une forme choisie dans le groupe consistant en un film, une mousse à alvéoles ouverts ou fermés, un revêtement non adhésif, une étoffe tissée, une étoffe non tissée, des fibres, des mailles, des tamis, des structures à effet de mèche, des structures perforées, des structures poreuses, des feuilles planes ou non planes, des structures planes ou non planes, et leurs combinaisons.

7. Dispositif de stomie selon l'une quelconque des revendications précédentes, dans lequel l'adhésif est une structure disposée en couches.

8. Dispositif de stomie selon l'une quelconque des revendications précédentes, dans lequel le soutien est une structure disposée en couches.

9. Dispositif de stomie selon l'une quelconque des revendications précédentes, comprenant en outre une doublure de libération pour protéger la surface adhésive avant utilisation.

10. Dispositif de stomie selon la revendication 8, dans lequel le dispositif est une structure non plane incluant des structures de forme concave ou convexe.

11. Dispositif de stomie selon la revendication 8, dans lequel le dispositif est sensiblement une structure plane.

12. Dispositif de stomie selon l'une quelconque des revendications précédentes, dans lequel ledit adhésif autocollant en silicone réticulé est un adhésif à base de solvant.

13. Dispositif de stomie selon l'une quelconque des revendications précédentes, dans lequel ledit adhésif autocollant en silicone réticulé sensible à la pression est un adhésif en gel de silicone.

14. Dispositif de stomie selon l'une quelconque des revendications précédentes, dans lequel ledit adhésif autocollant en silicone réticulé sensible à la pression est un adhésif en silicone durcissable.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2290974 A
- US 6840924 B
- US 4831070 A

- US 2004102744 A
- WO 03026541 A
- WO 9853772 A